# EUROPEAN PATENT APPLICATION

(11) **EP 3 932 306 A1**
(43) Date of publication of application: **05.01.2022**
(21) Application number: 19916860.0
(22) Date of filing: 28.02.2019
(51) Int. Cl.: A61B 5/107

(54) **ATTRIBUTE ESTIMATION DEVICE, ATTRIBUTE ESTIMATION METHOD, AND COMPUTER-READABLE RECORDING MEDIUM**

(71) Applicant: NEC Corporation, 108-8001 Tokyo (JP)
(72) Inventor: ARAKAWA, Takayuki, Tokyo 108-8001 (JP)
(74) Representative: Betten & Resch
(86) International application number: PCT/JP2019/007920
(87) International publication number: WO 2020/174682

(57) **Abstract**

An attribute estimation apparatus 1 includes: a generation unit 2 configured to generate acoustic characteristic information indicating an acoustic characteristic using a first acoustic signal output to an ear canal and a second acoustic signal produced by the first acoustic signal echoing inside the body; and an estimation unit 3 configured to estimate attributes using the acoustic characteristic information.

## Description

### TECHNICAL FIELD

The present invention relates to an attribute estimation apparatus and attribute estimation method for estimating attributes, and further relates to a computer readable recording medium that includes recorded thereon, a program for realizing the attribute estimation apparatus and attribute estimation method.

### BACKGROUND ART

Apparatuses are known for smoothly performing criminal investigation, marketing, and the like using a technique for estimating attributes of an individual. For example, the technique for estimating attributes of an individual is used for narrowing down suspects in criminal investigation based on results of performing matching between evidences remained in the crime scene and the attributes of individuals.

Also, the technique for estimating attributes of an individual is used, in marketing, for creating a model for estimating what kind of commercial product is purchased by a customer in what conditions, efficient product development, and planning the sales strategy based on results of performing matching between commercial products and the attributes of purchasers.

Also, as a related technique, Patent Document 1 discloses an apparatus that estimates attributes of an individual (gender, age) based on voice and video. With the apparatus, the gender and age are estimated using a feature value extracted from voice information and video information.

### LIST OF RELATED ART DOCUMENTS

### PATENT DOCUMENT

Patent Document 1: Japanese Patent Laid-Open Publication No. 2010-152866

### SUMMARY

### TECHNICAL PROBLEMS

However, in the apparatus disclosed in Patent Document 1 described above, user's voice is input using a microphone, and therefore the voice is likely to be influenced by noise such as environmental sound. Therefore, the accuracy in estimating attributes of an individual is degraded.

Also, the apparatus disclosed in Patent Document 1 estimates attributes of an individual by further combining an image in which user's expression is captured to the user's voice. However, the image is likely to be influenced by illumination and the like, and as a result, the accuracy in estimating attributes of an individual is degraded.

An example object of the invention is to provide an attribute estimation apparatus, an attribute estimation method, and a computer readable recording medium for estimating attributes of a subject using an echo signal.

### SOLUTION TO THE PROBLEMS

In order to achieve the above-described object, an attribute estimation apparatus according to an example aspect of the invention includes:
a generation unit configured to generate acoustic characteristic information indicating an acoustic characteristic using a first acoustic signal output to the ear canal and a second acoustic signal produced by the first acoustic signal echoing inside the body; and
an estimation unit configured to estimate attributes using the acoustic characteristic information.

In addition, in order to achieve the above-described object, an attribute estimation method according to an example aspect of the invention includes:
(a) a step of generating acoustic characteristic information indicating an acoustic characteristic using a first acoustic signal output to the ear canal and a second acoustic signal produced by the first acoustic signal echoing inside the body; and
(b) a step of estimating attributes using the acoustic characteristic information.

Furthermore, in order to achieve the above-described object, a computer readable recording medium that includes a program recorded thereon according to an example aspect of the invention includes recorded thereon, a program including instructions that cause a computer to carry out:
(a) a step of generating acoustic characteristic information indicating an acoustic characteristic using a first acoustic signal output to the ear canal and a second acoustic signal produced by the first acoustic signal echoing inside the body; and
(b) a step of estimating attributes using the acoustic characteristic information.

### ADVANTAGEOUS EFFECTS OF THE INVENTION

As described above, according to the invention, the attributes of a subject can be estimated using an echo signal.

### BRIEF DESCRIPTION OF THE DRAWINGS

FIG. 1 is a diagram illustrating an example of an attribute estimation apparatus.
FIG. 2 is a diagram illustrating an example of a system including the attribute estimation apparatus.
FIG. 3 is a diagram illustrating an example of resonance frequencies.
FIG. 4 is a diagram illustrating an example of the data structure of resonance frequency information.
FIG. 5 is a diagram for describing a learning model.
FIG. 6 is a diagram illustrating an example of the data structure of resonance frequency information in a modification.
FIG. 7 is a diagram for describing a learning model in the modification.
FIG. 8 is a diagram illustrating an example of the operations of the attribute estimation apparatus.
FIG. 9 is a diagram illustrating an example of a computer for realizing the attribute estimation apparatus.

### EXAMPLE EMBODIMENT

### (Example Embodiment)

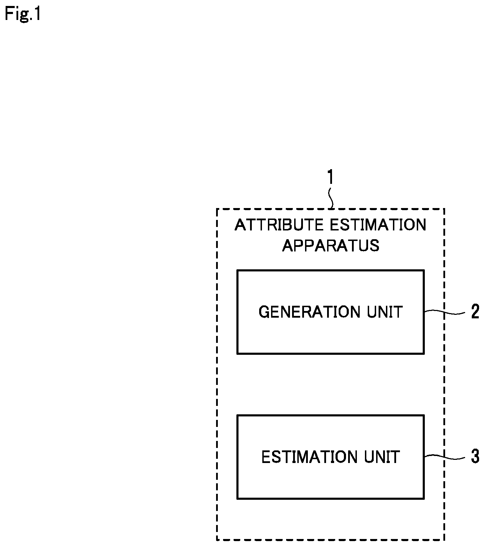

In the following, an example embodiment of the invention will be described with reference to FIGS. 1 to 9.

### [Apparatus configuration]

First, a configuration of an attribute estimation apparatus 1 in the example embodiment will be described with reference to FIG. 1. FIG. 1 is a diagram illustrating one example of the attribute estimation apparatus.

The attribute estimation apparatus 1 shown in FIG. 1 is an apparatus that can estimate attributes of a subject using an echo signal. Also, as shown in FIG. 1, the attribute estimation apparatus 1 includes a generation unit 2 and an estimation unit 3.

Of the two units, the generation unit 2 generates acoustic characteristic information indicating an acoustic characteristic using an acoustic signal (first acoustic signal) output to the ear canal and an echo signal (second acoustic signal) produced by the acoustic signal echoing inside the body. The estimation unit 3 estimates attributes using the acoustic characteristic information.

In such a manner, in the example embodiment, acoustic characteristic information such as an impulse response h(t) or a transfer function H(ω) or H(z) is generated using an acoustic signal x(t) output to the ear canal of a target user and an echo signal y(t) reflecting the states of organs inside the body. Therefore, the states of organs inside the body can be estimated from the acoustic characteristic information, and as a result, the attributes such as gender, age, body length, body weight, body build (body shape, skeletal frame shape, skeletal frame size) of a subject can be accurately estimated.

### [System configuration]

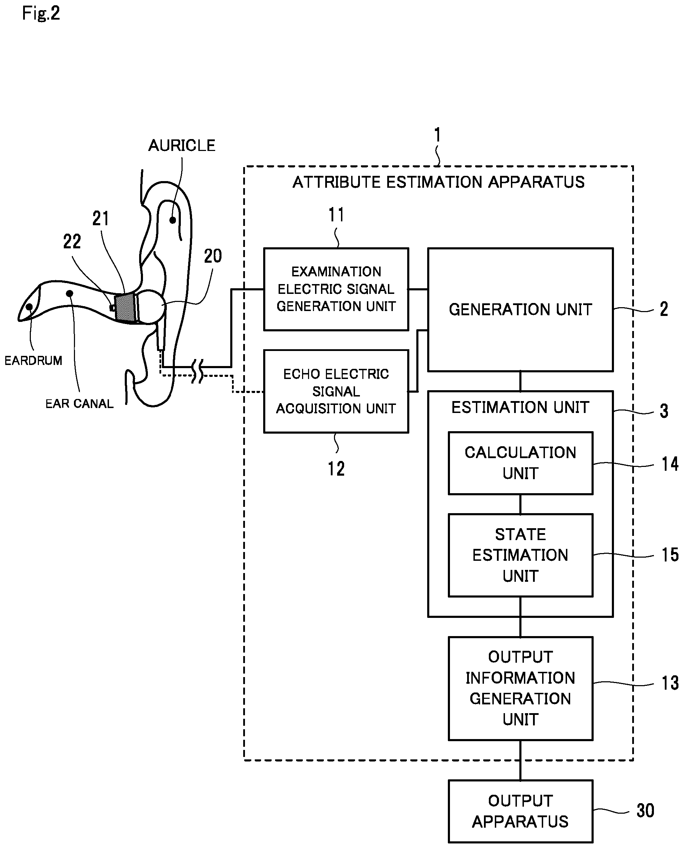

Next, the configuration of the attribute estimation apparatus 1 in the example embodiment will be described in detail with reference to FIG. 2. FIG. 2 is a diagram illustrating one example of a system including the attribute estimation apparatus.

As illustrated in FIG. 2, the system in the example embodiment includes an ear-mounted apparatus 20 and an output apparatus 30, in addition to the attribute estimation apparatus 1. Furthermore, the attribute estimation apparatus 1 includes an examination electric signal generation unit 11, an echo electric signal acquisition unit 12, and an output information generation unit 13, in addition to the generation unit 2 and the estimation unit 3. Furthermore, the estimation unit 3 includes a calculation unit 14, and an attribute estimation unit 15. The ear-mounted apparatus 20 includes an examination sound signal reproduction unit 21 and an echo sound signal recording unit 22.

The ear-mounted apparatus 20 includes the examination sound signal reproduction unit 21, which is for outputting an acoustic signal to the ear canal, and the echo sound signal recording unit 22, which is for receiving input of (measuring) an echo signal in the ear canal. Specifically, the ear-mounted apparatus 20 is an apparatus that is used in a state in which the ear-mounted apparatus 20 is worn in the ear canal, as illustrated in the cross-sectional diagram of the outer ear (diagram illustrating the auricle, ear canal, and eardrum) in FIG. 2. For example, an earphone provided with a microphone is conceivable as the ear-mounted apparatus 20.

Note that the configuration of the ear-mounted apparatus 20 is not limited to that illustrated in FIG. 2, and any configuration may be adopted as long as an echo signal corresponding to an acoustic signal can be measured.

Upon receiving an electric signal generated by the examination electric signal generation unit 11 that corresponds to an acoustic signal, the examination sound signal reproduction unit 21 (acoustic signal output unit) generates the acoustic signal based on the received electric signal and outputs the generated acoustic signal to the ear canal. Note that a speaker or the like, for example, is conceivable as the examination sound signal reproduction unit 21.

Upon receiving an echo signal corresponding to the acoustic signal output from the examination electric signal generation unit 11, the echo sound signal recording unit 22 (acoustic signal input unit) converts the echo signal into an electric signal and transmits the electric signal to the echo electric signal acquisition unit 12. Note that a microphone or the like, for example, is conceivable as the echo sound signal recording unit 22.

The output apparatus 30 acquires the later-described output information, which has been converted into an outputtable format by the output information generation unit 13, and outputs images, sounds, etc., generated based on the output information. The output apparatus 30 is an image display device, etc., in which liquid crystal, organic electro-luminescence (EL), or a cathode ray tube (CRT) is used, for example. Furthermore, the image display device may include a sound output device such as a speaker. Note that the output apparatus 30 may be a printing device such as a printer.

The examination electric signal generation unit 11 generates the electric signal used to output the acoustic signal, and transmits the electric signal to the examination sound signal reproduction unit 21. Specifically, the examination electric signal generation unit 11 generates, as the electric signal corresponding to the acoustic signal, a maximal length sequence (M-sequence) signal, a time-stretched pulse (TSP) signal, a Log-TSP signal, or the like. Furthermore, the examination electric signal generation unit 11 transmits the electric signal corresponding to the acoustic signal to the generation unit 2.

Note that a sweep signal, music, audio guidance, etc., may be included in the acoustic signal. Furthermore, the frequencies used for the acoustic signal are set in accordance with target organs. For example, when the vocal and respiratory tracts, etc., are set as targets, the frequency band of the acoustic signal is preferably set to 100-4k [Hz]. However, there is no limitation to this frequency band.

Here, the vocal tract (articulatory organs), for example, is a path of voice, and is a cavity in the body through which sound produced by the vocal cords passes before being emitted to the outside of the body. The respiratory tract (phonatory organs), for example, is a path of respiratory sound and is involved in external respiration. The respiratory tract is formed from the upper respiratory tract (the nasal cavity, the pharynx, the larynx, etc.) and the lower respiratory tract (the trachea, the primary bronchi, the lungs, etc.).

The echo electric signal acquisition unit 12 receives the electric signal corresponding to the echo signal from the echo sound signal recording unit 22, adjusts the received electric signal, and transmits the adjusted electric signal to the generation unit 2. Specifically, the echo electric signal acquisition unit 12 adjusts the received electric signal using a circuit including a filter, an amplifier, etc., and transmits the adjusted electric signal to the generation unit 2.

The generation unit 2 generates acoustic characteristic information indicating an acoustic characteristic using an electric signal corresponding to an acoustic signal x(t) and an electric signal corresponding to an echo signal y(t). For example, an impulse response h(t), a transfer function H(co) or H(z) obtained by performing Fourier transform or Laplace transform on the impulse response, or the like is used as the acoustic characteristic.

Specifically, the generation unit 2 first receives the electric signal corresponding to the acoustic signal x(t) from the examination electric signal generation unit 11. Furthermore, the generation unit 2 receives the electric signal corresponding to the echo signal y(t) from the echo electric signal acquisition unit 12. Subsequently, the generation unit 2 generates the acoustic characteristic information (an impulse response h(t), a transfer function H(ω) or H(z), or the like) based on the received electric signals corresponding to the acoustic signal x(t) and the echo signal y(t).

Subsequently, the generation unit 2 stores the acoustic characteristic information to a storage unit, which is not illustrated. Note that the storage unit may be provided inside or outside the attribute estimation apparatus 1.

Since the echo signal y(t) reflects changes (changes in reflection ratio, attenuation rate, etc.) that are in accordance with the states of the subject's organs, information relating to the states of organs inside the body can be extracted by generating the acoustic characteristic information, which is an impulse response h(t), a transfer function H(ω) or H(z), or the like. Note that the reflection ratio is the ratio of the reflection to the input, and the attenuation rate is the rate of attenuation per unit time or unit cycle.

Note that, the echo signal includes an acoustic signal coming back from spaces (the ear canal, and the vocal and respiratory tracts) located between the head and the lungs, for example.

The estimation unit 3 estimates the shapes of ear canal, vocal tract, and respiratory tract using the acoustic characteristic information, and estimates attributes using the estimated shapes of the ear canal shape, vocal tract, and respiratory tract. The attributes include gender, age, body length, body weight, and body build, for example.

Specifically, the estimation unit 3 calculates resonance frequency information indicating the resonance frequency with respect to each of the ear canal, vocal tract, and respiratory tract using the acoustic characteristic information, and estimates the shapes of the ear canal, vocal tract, respiratory tract using the calculated resonance frequency information.

Moreover, the estimation unit 3 may also calculate the attenuation coefficient with respect to each resonance frequency information, and estimate the shapes of the ear canal, vocal tract, and respiratory tract using the resonance frequency information and the attenuation coefficients.

The estimation unit 3 (calculation unit 14, attribute estimation unit 15) will be described in detail.

The calculation unit 14 calculates resonance frequency information including information indicating the resonance frequency (frequency of peak value in frequency characteristic) for each organ type using the acoustic characteristic information.

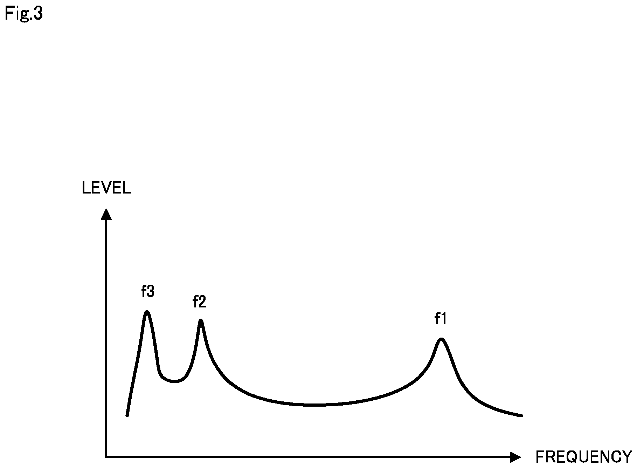

FIG. 3 is a diagram illustrating an example of resonant frequencies. FIG. 3 illustrates resonant frequencies f1, f2, and f3 included in resonant frequency information.

The calculation of resonant frequencies will be described.

The calculation unit 14 first acquires acoustic characteristic information from the generation unit 2. Next, the calculation unit 14 calculates the resonance frequencies regarding the ear canal, vocal tract, and respiratory tract using the acoustic characteristics. The calculation unit 14 calculates resonance frequencies by adopting linear predictive coding (LPC) or the like, for example. Thereafter, the calculation unit 14 generates resonance frequency information indicating the resonance frequencies, and stores the generated resonance frequency information in the storage unit. Note that the method for calculating resonant frequencies is not limited to the linear predictive coding, and any method may be used as long as resonant frequencies can be calculated.

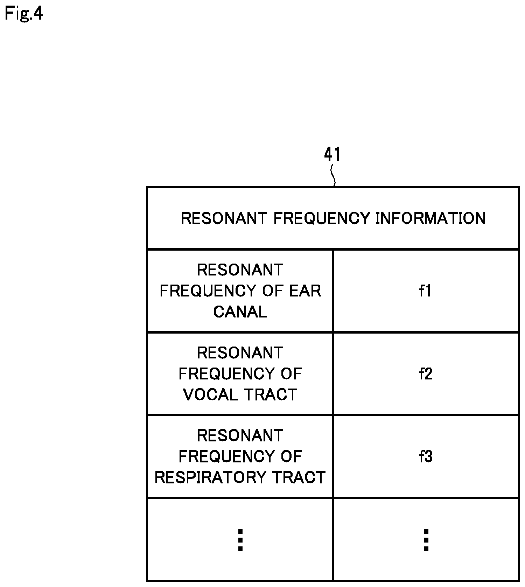

FIG. 4 is a diagram illustrating an example of the data structure of the resonance frequency information. The resonance frequency information 41 in FIG. 4 includes a resonance frequency f1 corresponding to the ear canal, a resonance frequency f2 corresponding to the vocal tract, and a resonance frequency f3 corresponding to the respiratory tract, the resonance frequencies being calculated by the calculation unit 14.

For example, when the length of ear canal is about 2.8 [cm], the length of vocal tract is about 8.5 [cm], the length of respiratory tract is about 17.0 [cm], and the sound velocity is 340 [m/sec], the resonance frequency f1 appears in the vicinity of 6k [Hz], the resonance frequency f2 appears in the vicinity of 1k [Hz], and the resonance frequency f3 appears in the vicinity of 500 [Hz].

The reason is because, in an air column pipe resonant model, as the length of pipe decreases, the frequency increases, and as the length of pipe increases, the frequency decreases.

The attribute estimation unit 15 estimates attributes using the calculated resonance frequency information. Specifically, the attribute estimation unit 15 first acquires the generated resonance frequency information. Next, the attribute estimation unit 15 estimates attributes such as gender, age, body length, body weight, and body build using a learning model for estimating attributes, using the acquired resonance frequency information (resonance frequencies f1 to f3 of respective organs) as inputs, for example. Thereafter, the attribute estimation unit 15 transmits information indicating the estimated attributes to the output information generation unit 13.

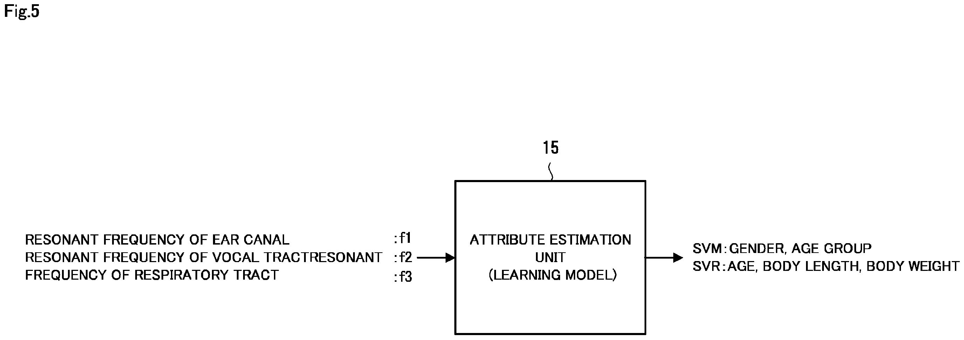

FIG. 5 is a diagram for describing a learning model. As shown in FIG. 5, by inputting the resonance frequency f1 corresponding to the ear canal, the resonance frequency f2 corresponding to the vocal tract, and the resonance frequency f3 corresponding to the respiratory tract to the learning model of the attribute estimation unit 15, attributes that are the result of estimation are obtained.

It is conceivable that SVM (Support Vector Machine) is used as the learning model when estimating attributes as an identification problem, and SVR (Support Vector Regression) is used as the earning model when estimating attributes as a regression problem, for example.

Moreover, the learning model in which SVM is used is preferable for determining the gender (male/female) and the age group (less than ten, ten to nineteen, twenties, etc.). Also, the learning model in which SVR is used is preferable for determining the age, body length, body weight, body build, and the like. Note that the learning model is not limited to SVM and SVR

Note that the learning model is generated by performing learning using supervised data in a learning phase, in advance.

The output information generation unit 13, upon acquiring information indicating the attributes from the attribute estimation unit 15, generates output information based on the information, and transmits the output information to the output apparatus 30. The output apparatus 30 outputs the attributes of the subject based on the output information.

### [Modification]

A modification will be described. In the modification, the attributes are estimated using a resonance frequency and an attenuation coefficient corresponding to the resonance frequency. With this, the number of attributes that can be estimated can be increased.

For example, when the attenuation coefficient is large, it is known that it is highly possible that the subject is thin. The reason is because, when the attenuation coefficient is large, the attenuation is slow, and it is estimated that the acoustic signal is reflected on a hard face. That is, the ratio of bone is large relative to fat and muscle, regarding the subject, and therefore it can be estimated that it is highly possible that the subject is thin.

The calculation unit 14 in the modification will be described.

The calculation unit 14 calculates resonance frequency information including a resonance frequency and an attenuation coefficient for each organ type using the acoustic characteristic information. Specifically, the calculation unit 14 calculates resonance frequencies and attenuation coefficients corresponding to the respective resonance frequencies. It is conceivable to calculate a damping ratio (damping factor), a logarithmic decrement, a loss factor, or a Q-value, and use the calculated item as the attenuation coefficient.

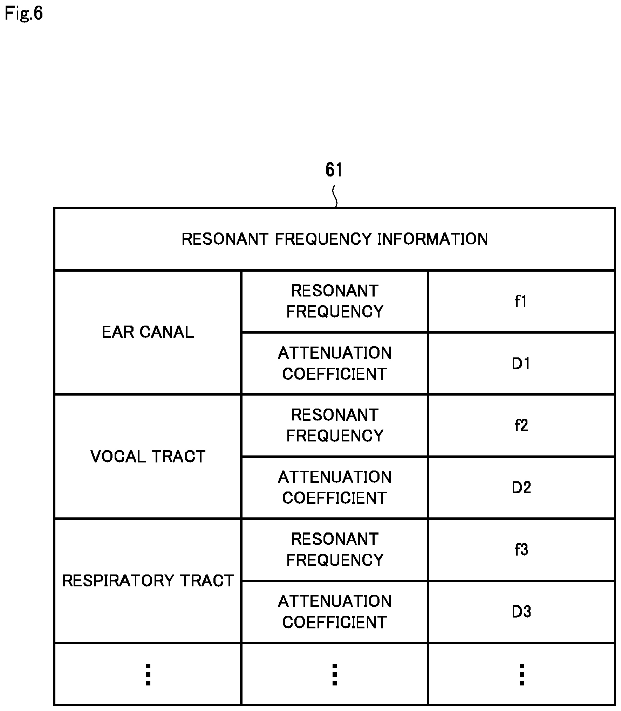

FIG. 6 is a diagram illustrating an example of the data structure of the resonance frequency information in the modification. In the resonance frequency information in FIG. 6, the resonance frequencies f1, f2, and f3 are respectively associated with pieces of information D1, D2, and D3 indicating attenuation coefficients.

The attribute estimation unit 15 in the modification will be described.

The attribute estimation unit 15 estimates attributes using resonance frequency information including the calculated attenuation coefficients. Specifically, the attribute estimation unit 15 first acquires resonance frequency information including the generated attenuation coefficients.

Next, the attribute estimation unit 15 estimates attributes such as gender, age, body length, body weight, and body build using a learning model for estimating the attributes, using the resonance frequency information as shown in FIG. 6 as inputs, for example. Thereafter, the attribute estimation unit 15 transmits information indicating the estimated attributes to the output information generation unit 13.

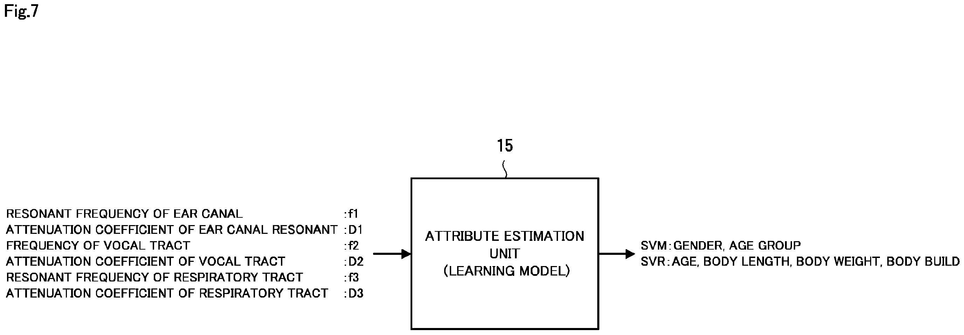

FIG. 7 is a diagram for describing the learning model in the modification. As shown in FIG. 7, by inputting the resonance frequency f1 and the attenuation coefficient D1 corresponding to the ear canal, the resonance frequency f2 and the attenuation coefficient D2 corresponding to the vocal tract, and the resonance frequency f3 and the attenuation coefficient D3 corresponding to the respiratory tract to the learning model of the attribute estimation unit 15, attributes that are the result of estimation are obtained.

### [Apparatus operations]

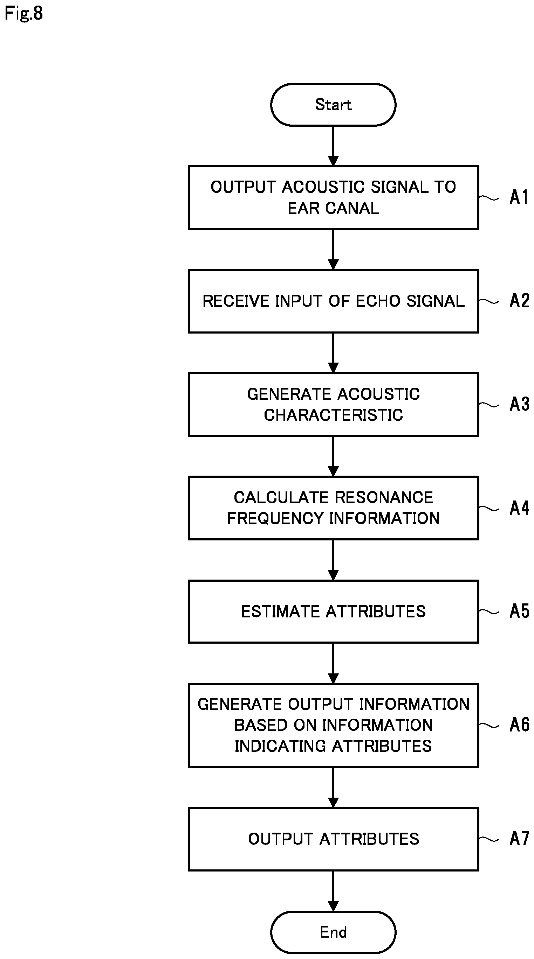

Next, operations of the attribute estimation apparatus in the example embodiment of the invention will be described with reference to FIG. 8. FIG. 8 is a diagram illustrating an example of the operations of the attribute estimation apparatus. FIGS. 2 to 7 will be referred to as needed in the following description. Furthermore, in the example embodiment, an attribute estimation method is implemented by causing the attribute estimation apparatus to operate. Accordingly, the following description of the operations of the attribute estimation apparatus is substituted for the description of the attribute estimation method in the example embodiment.

As shown in FIG. 8, first, the examination sound signal reproduction unit 21, upon receiving an electric signal corresponding to the acoustic signal generated by the examination electric signal generation unit 11, generates an acoustic signal based on the received electric signal, and outputs the generated acoustic signal to the ear canal (step A1).

Next, the echo sound signal recording unit 22 receives (measures) an echo signal with respect to the acoustic signal output from the examination electric signal generation unit 11 (step A2). Thereafter, the echo sound signal recording unit 22 converts the received echo signal to an electric signal, and transmits the electric signal to the echo electric signal acquisition unit 12.

Next, the generation unit 2 generates acoustic characteristic information indicating an acoustic characteristic using an electric signal corresponding to an acoustic signal x(t) and an electric signal corresponding to an echo signal y(t) (step A3). For example, an impulse response h(t), a transfer function H(ω) or H(z) obtained by performing Fourier transform or Laplace transform on the impulse response, or the like is used as the acoustic characteristic.

Specifically, in step A3, the generation unit 2 first receives an electric signal corresponding to an acoustic signal x(t) from the examination electric signal generation unit 11. Also, the generation unit 2 receives an electric signal corresponding to an echo signal y(t) from the echo electric signal acquisition unit 12.

Next, in step A3, the generation unit 2 generates acoustic characteristic information (impulse response h(t), transfer function H(ω) or H(z), etc.) based on the received electric signals corresponding to the acoustic signal x(t) and echo signal y(t). Then, in step A3, the generation unit 2 stores the acoustic characteristic information in a storage unit that is not illustrated.

Next, the calculation unit 14 calculates resonance frequency information indicating the resonance frequency for each organ type using the acoustic characteristic information (step A4).

The calculation of the resonance frequency in step A4 will be described.

In step A4, the calculation unit 14 first acquires the acoustic characteristic information from the generation unit 2. Subsequently, the calculation unit 14 performs spectral analysis using the acoustic characteristic, and calculates resonant frequencies regarding the subject. The calculation unit 14 calculates resonant frequencies using linear predictive coding (LPC), etc., for example. Then, in step A4, the calculation unit 14 generates resonant frequency information indicating the resonant frequencies, and stores the generated resonant frequency information in the storage unit. Note that the method for calculating resonant frequencies is not limited to linear predictive coding, and any method may be used as long as resonant frequencies can be calculated.

Next, the attribute estimation unit 15 estimates attributes using the calculated resonance frequency information (step A5). Specifically, in step A5, the attribute estimation unit 15 first acquires the generated resonance frequency information.

Next, in step A5, the attribute estimation unit 15 estimates attributes such as gender, age, body length, body weight, and body build using a learning model for identifying attributes, using the acquired resonance frequency information (resonance frequencies f1 to f3 of respective organs) as inputs, for example.

Thereafter, in step A5, the attribute estimation unit 15 transmits information indicating the estimated attributes to the output information generation unit 13.

Subsequently, upon acquiring the information indicating the attributes from the attribute estimation unit 15, the output information generation unit 13 generates output information based on the information (step A6). Furthermore, the output information generation unit 13 transmits the output information to the output apparatus 30. Subsequently, the output apparatus 30 outputs the attributes of the subject based on the output information (step A7).

### [Modification]

Operations in the modification will be described. In the modification, the attributes are estimated using resonance frequencies and attenuation coefficients corresponding to the resonance frequencies. With this, the number of attributes that can be estimated can be increased.

### The calculation unit 14 in the modification will be described.

In step A4, the calculation unit 14 calculates resonance frequency information including the resonance frequency and the attenuation coefficient for each organ type using the acoustic characteristic information. Specifically, in step A4, the calculation unit 14 calculates resonance frequencies and attenuation coefficients corresponding to the respective resonance frequencies. It is conceivable to calculate a damping ratio (damping factor), a logarithmic decrement, a loss factor, or a Q-value, and use the calculated item as the attenuation coefficient.

### The attribute estimation unit 15 in the modification will be described.

In step A5, the attribute estimation unit 15 estimates attributes using the resonance frequency information including the calculated attenuation coefficients. Specifically, in step A5, the attribute estimation unit 15 first acquires the resonance frequency information including the generated attenuation coefficients.

Next, in step A5, the attribute estimation unit 15 estimates attributes such as gender, age, body length, body weight, and body build using the learning model for identifying the attributes, using the resonance frequency information as shown in FIG. 6 as inputs, for example. Thereafter, in step A5, the attribute estimation unit 15 transmits information indicating the estimated attributes to the output information generation unit 13.

### [Effects of example embodiment]

As described above, according to the example embodiment, acoustic characteristic information such as an impulse response h(t) or a transfer function H(z) is generated using an acoustic signal x(t) output to the ear canal of a target user and an echo signal y(t) reflecting the states of organs inside the body. Therefore, because the states of organs inside the body can be estimated from the acoustic characteristic information, the attributes of the subject such as gender, age, body length, body weight, and body build can be estimated.

Also, the attributes of a subject can be estimated using the example embodiment, and therefore the present invention is useful in fields such as criminal investigation and marketing.

### [Program]

It suffices for a program in the example embodiment of the invention to be a program that causes a computer to carry out steps A1 to A8 illustrated in FIG. 8. By installing this program on a computer and executing the program, the attribute estimation apparatus and the attribute estimation method in the example embodiment can be realized. In this case, the processor of the computer functions and performs processing as the generation unit 2, the estimation unit 3 (the calculation unit 14 and the attribute estimation unit 15), and the output information generation unit 13.

Furthermore, the program in the example embodiment may be executed by a computer system formed from a plurality of computers. In this case, the computers may each function as one of the generation unit 2, the estimation unit 3 (the calculation unit 14 and the attribute estimation unit 15), and the output information generation unit 13, for example.

### [Physical configuration]

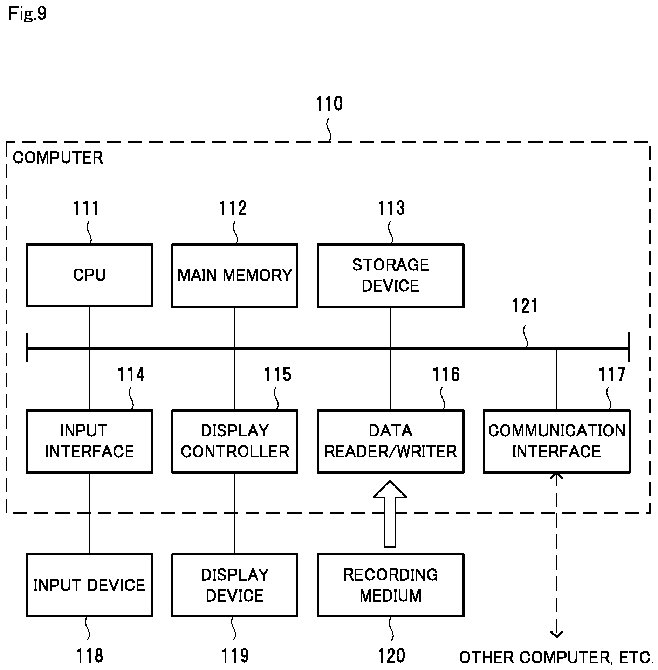

Here, a computer that realizes the attribute estimation apparatus by executing the program in the example embodiment will be described with reference to FIG. 9. FIG. 9 is a block diagram illustrating one example of a computer realizing the attribute estimation apparatus in the example embodiment of the invention.

As illustrated in FIG. 9, a computer 110 includes a CPU 111, a main memory 112, a storage device 113, an input interface 114, a display controller 115, a data reader/writer 116, and a communication interface 117. These components are connected via a bus 121 so as to be capable of performing data communication with one another. Note that the computer 110 may include a graphics processing unit (GPU) or a field-programmable gate array (FPGA) in addition to the CPU 111 or in place of the CPU 111.

The CPU 111 loads the program (codes) in the example embodiment, which is stored in the storage device 113, onto the main memory 112, and performs various computations by executing these codes in a predetermined order. The main memory 112 is typically a volatile storage device such as a dynamic random access memory (DRAM). Furthermore, the program in the example embodiment is provided in a state such that the program is stored in a computer readable recording medium 120. Note that the program in the example embodiment may also be a program that is distributed on the Internet, to which the computer 110 is connected via the communication interface 117.

In addition, specific examples of the storage device 113 include semiconductor storage devices such as a flash memory, in addition to hard disk drives. The input interface 114 mediates data transmission between the CPU 111 and input equipment 118 such as a keyboard and a mouse. The display controller 115 is connected to a display device 119, and controls the display performed by the display device 119.

The data reader/writer 116 mediates data transmission between the CPU 111 and the recording medium 120, and executes the reading out of the program from the recording medium 120 and the writing of results of processing in the computer 110 to the recording medium 120. The communication interface 117 mediates data transmission between the CPU 111 and other computers.

Furthermore, specific examples of the recording medium 120 include a general-purpose semiconductor storage device such as a CompactFlash (registered trademark, CF) card or a Secure Digital (SD) card, a magnetic recording medium such as a flexible disk, and an optical recording medium such as a compact disk read-only memory (CD-ROM).

Note that the attribute estimation apparatus 1 in the example embodiment can also be realized by using pieces of hardware corresponding to the respective units, rather than using a computer on which the program is installed. Furthermore, a portion of the attribute estimation apparatus 1 may be realized by using a program, and the remaining portion of the emotion estimation apparatus 1 may be realized by using hardware.

### [Supplementary note]

In relation to the above example embodiment, the following Supplementary notes are further disclosed. While a part of or the entirety of the above-described example embodiment can be expressed by (Supplementary note 1) to (Supplementary note 16) described in the following, the invention is not limited to the following description.

### (Supplementary note 1)

An attribute estimation apparatus comprising:
a generation unit configured to generate acoustic characteristic information indicating an acoustic characteristic using a first acoustic signal output to the ear canal and a second acoustic signal produced by the first acoustic signal echoing inside the body; and
an estimation unit configured to estimate attributes using the acoustic characteristic information.

### (Supplementary note 2)

The attribute estimation apparatus according to Supplementary note 1, wherein
the estimation unit estimates the shape of the ear canal, the shape of a vocal tract, and the shape of a respiratory tract using the acoustic characteristic information, and estimates the attributes using the estimated shapes of the ear canal, the vocal tract, and the respiratory tract.

### (Supplementary note 3)

The attribute estimation apparatus according to Supplementary note 2, wherein
the estimation unit calculates resonance frequency information indicating the resonance frequency with respect to each of the ear canal, the vocal tract, and the respiratory tract, using the acoustic characteristic information, and estimates the shapes of the ear canal, the vocal tract, and the respiratory tract using the calculated resonance frequency information.

### (Supplementary note 4)

The attribute estimation apparatus according to Supplementary note 3, wherein
the estimation unit calculates an attenuation coefficient with respect to each resonance frequency information, and estimates the shapes of the ear canal, the vocal tract, and the respiratory tract using the resonance frequency information and the attenuation coefficients.

### (Supplementary note 5)

The attribute estimation apparatus according to any one of Supplementary notes 1 to 4, wherein
the attributes include at least one or more of gender, age, body length, body weight, and body build.

### (Supplementary note 6)

The attribute estimation apparatus according to any one of Supplementary notes 1 to 5, further comprising:
an acoustic signal output unit configured to output the first acoustic signal to the ear canal; and
an acoustic signal input unit configured to receive input of the second acoustic signal.

### (Supplementary note 7)

An attribute estimation method comprising:
(a) a step of generating acoustic characteristic information indicating an acoustic characteristic using a first acoustic signal output to an ear canal and a second acoustic signal produced by the first acoustic signal echoing inside the body; and
(b) a step of estimating attributes using the acoustic characteristic information.

### (Supplementary note 8)

The attribute estimation method according to Supplementary note 7, wherein
in the (b) step, the shapes of the ear canal, a vocal tract, and a respiratory tract are estimated using the acoustic characteristic information, and the attributes are estimated using the estimated shapes of the ear canal, the vocal tract, and the respiratory tract.

### (Supplementary note 9)

The attribute estimation method according to Supplementary note 8, wherein
in the (b) step, resonance frequency information indicating the resonance frequency is calculated with respect to each of the ear canal, the vocal tract, and the respiratory tract, using the acoustic characteristic information, and the shapes of the ear canal, the vocal tract, and the respiratory tract are estimated using the calculated resonance frequency information.

### (Supplementary note 10)

The attribute estimation method according to Supplementary note 9, wherein
in the (b) step, an attenuation coefficient is calculated with respect to each resonance frequency information, and the shapes of the ear canal, the vocal tract, and the respiratory tract are estimated using the resonance frequency information and the attenuation coefficients.

### (Supplementary note 11)

The attribute estimation method according to any one of Supplementary notes 7 to 10, wherein
the attributes include at least one or more of gender, age, body length, body weight, and body build.

### (Supplementary note 12)

A computer readable recording medium that includes recorded thereon, a program including instructions that cause a computer to carry out:
(a) a step of generating acoustic characteristic information indicating an acoustic characteristic using a first acoustic signal output to an ear canal and a second acoustic signal produced by the first acoustic signal echoing inside the body; and
(b) a step of estimating attributes using the acoustic characteristic information.

### (Supplementary note 13)

The computer readable recording medium according to Supplementary note 12, wherein
in the (b) step, the shapes of the ear canal, a vocal tract, and a respiratory tract are estimated using the acoustic characteristic information, and the attributes are estimated using the estimated shapes of the ear canal, the vocal tract, and the respiratory tract.

### (Supplementary note 14)

The computer readable recording medium according to Supplementary note 13, wherein
in the (b) step, resonance frequency information indicating the resonance frequency is calculated with respect to each of the ear canal, the vocal tract, and the respiratory tract, using the acoustic characteristic information, and the shapes of the ear canal, the vocal tract, and the respiratory tract are estimated using the calculated resonance frequency information.

### (Supplementary note 15)

The computer readable recording medium according to Supplementary note 14, wherein
in the (b) step, an attenuation coefficient is calculated with respect to each resonance frequency information, and the shapes of the ear canal, the vocal tract, and the respiratory tract are estimated using the resonance frequency information and the attenuation coefficients.

### (Supplementary note 16)

The computer readable recording medium according to any one of Supplementary notes 12 to 15, wherein
the attributes include at least one or more of gender, age, body length, body weight, and body build.

The invention has been described with reference to an example embodiment above, but the invention is not limited to the above-described example embodiment. Within the scope of the invention, various changes that could be understood by a person skilled in the art could be applied to the configurations and details of the invention.

### INDUSTRIAL APPLICABILITY

As described above, according to the invention, the attributes of a subject can be estimated using an echo signal. The invention is useful in fields such as criminal investigation and marketing.

### REFERENCE SIGNS LIST

- 1: Attribute estimation apparatus
- 2: Generation unit
- 3: Estimation unit
- 11: Examination electric signal generation unit
- 12: Echo electric signal acquisition unit
- 13: Output information generation unit
- 14: Calculation unit
- 15: Attribute estimation unit
- 20: Ear-mounted apparatus
- 21: Examination sound signal reproduction unit
- 22: Echo sound signal recording unit
- 30: Output apparatus
- 41, 61: Resonant frequency information
- 110: Computer
- 111: CPU
- 112: Main memory
- 113: Storage device
- 114: Input interface
- 115: Display controller
- 116: Data reader/writer
- 117: Communication interface
- 118: Input equipment
- 119: Display device
- 120: Recording medium
- 121: Bus

## Claims

1. An attribute estimation apparatus comprising:
a generation means for generating acoustic characteristic information indicating an acoustic characteristic using a first acoustic signal output to the ear canal and a second acoustic signal produced by the first acoustic signal echoing inside the body; and
an estimation means for estimating attributes using the acoustic characteristic information.

2. The attribute estimation apparatus according to claim 1, wherein
the estimation means estimates the shape of the ear canal, the shape of a vocal tract, and the shape of a respiratory tract using the acoustic characteristic information, and estimates the attributes using the estimated shapes of the ear canal, the vocal tract, and the respiratory tract.

3. The attribute estimation apparatus according to claim 2, wherein
the estimation means calculates resonance frequency information indicating the resonance frequency with respect to each of the ear canal, the vocal tract, and the respiratory tract, using the acoustic characteristic information, and estimates the shapes of the ear canal, the vocal tract, and the respiratory tract using the calculated resonance frequency information.

4. The attribute estimation apparatus according to claim 3, wherein
the estimation means calculates an attenuation coefficient with respect to each resonance frequency information, and estimates the shapes of the ear canal, the vocal tract, and the respiratory tract using the resonance frequency information and the attenuation coefficients.

5. The attribute estimation apparatus according to any one of claims 1 to 4, wherein
the attributes include at least one or more of gender, age, body length, body weight, and body build.

6. The attribute estimation apparatus according to any one of claims 1 to 5, further comprising:
an acoustic signal output means for outputting the first acoustic signal to the ear canal; and
an acoustic signal input means for receiving input of the second acoustic signal.

7. An attribute estimation method comprising:
(a) a step of generating acoustic characteristic information indicating an acoustic characteristic using a first acoustic signal output to an ear canal and a second acoustic signal produced by the first acoustic signal echoing inside the body; and
(b) a step of estimating attributes using the acoustic characteristic information.

8. The attribute estimation method according to claim 7, wherein
in the (b) step, the shapes of the ear canal, a vocal tract, and a respiratory tract are estimated using the acoustic characteristic information, and the attributes are estimated using the estimated shapes of the ear canal, the vocal tract, and the respiratory tract.

9. The attribute estimation method according to claim 8, wherein
in the (b) step, resonance frequency information indicating the resonance frequency is calculated with respect to each of the ear canal, the vocal tract, and the respiratory tract, using the acoustic characteristic information, and the shapes of the ear canal, the vocal tract, and the respiratory tract are estimated using the calculated resonance frequency information.

10. The attribute estimation method according to claim 9, wherein
in the (b) step, an attenuation coefficient is calculated with respect to each resonance frequency information, and the shapes of the ear canal, the vocal tract, and the respiratory tract are estimated using the resonance frequency information and the attenuation coefficients.

11. The attribute estimation method according to any one of claims 7 to 10, wherein
the attributes include at least one or more of gender, age, body length, body weight, and body build.

12. A computer readable recording medium that includes recorded thereon, a program including instructions that cause a computer to carry out:
(a) a step of generating acoustic characteristic information indicating an acoustic characteristic using a first acoustic signal output to an ear canal and a second acoustic signal produced by the first acoustic signal echoing inside the body; and
(b) a step of estimating attributes using the acoustic characteristic information.

13. The computer readable recording medium according to claim 12, wherein
in the (b) step, the shapes of the ear canal, a vocal tract, and a respiratory tract are estimated using the acoustic characteristic information, and the attributes are estimated using the estimated shapes of the ear canal, the vocal tract, and the respiratory tract.

14. The computer readable recording medium according to claim 13, wherein
in the (b) step, resonance frequency information indicating the resonance frequency is calculated with respect to each of the ear canal, the vocal tract, and the respiratory tract, using the acoustic characteristic information, and the shapes of the ear canal, the vocal tract, and the respiratory tract are estimated using the calculated resonance frequency information.

15. The computer readable recording medium according to claim 14, wherein
in the (b) step, an attenuation coefficient is calculated with respect to each resonance frequency information, and the shapes of the ear canal, the vocal tract, and the respiratory tract are estimated using the resonance frequency information and the attenuation coefficients.

16. The computer readable recording medium according to any one of claims 12 to 15, wherein
the attributes include at least one or more of gender, age, body length, body weight, and body build.
